(19) Europäisches Patentamt
European Patent Office
Office européen des brevets

(11) EP 1 391 729 A1

(12) **EUROPEAN PATENT APPLICATION**
published in accordance with Art. 158(3) EPC

(43) Date of publication:
**25.02.2004 Bulletin 2004/09**

(21) Application number: **02724687.5**

(22) Date of filing: **02.05.2002**

(51) Int Cl.[7]: **G01N 33/50**, G01N 33/15, A61K 39/00

(86) International application number:
**PCT/JP2002/004406**

(87) International publication number:
**WO 2002/090981 (14.11.2002 Gazette 2002/46)**

(84) Designated Contracting States:
**AT BE CH CY DE DK ES FI FR GB GR IE IT LI LU MC NL PT SE TR**

(30) Priority: **08.05.2001 JP 2001137526**

(71) Applicant: **JAPAN SCIENCE AND TECHNOLOGY CORPORATION**
**Kawaguchi-shi, Saitama 332-0012 (JP)**

(72) Inventors:
- **HANABUCHI, Shino,**
**Dept of Immuno. Tokyo M & D U.**
**Bunkyo-ku, Tokyo 113-8519 (JP)**
- **OHASHI, Takashi,**
**Dept of Immuno. Tokyo M & D U.**
**Bunkyo-ku, Tokyo 113-8519 (JP)**
- **KANNAGI, Mari, Dept of Immuno. Tokyo M & D U.**
**Bunkyo-ku, Tokyo 113-8519 (JP)**

(74) Representative: **Harding, Charles Thomas**
**D. Young & Co.**
**21 New Fetter Lane**
**London EC4A 1DA (GB)**

# (54) ANTITUMOR ANTIGEN AGAINST HTLV-1 TUMOR OR ANTIGEN EPITOPE THEREOF

(57) The present invention provides a vaccine for inducing an immune response containing a CTL recognition antigen or an antigen epitope thereof, which can be obtained by screening a CTL recognition antigen or an antigen epitope thereof having an anti-tumor effect against HTLV-I tumors such as ATL, or a DNA that encodes them as an active ingredient and the like. Dominant epitope GAFLTNVPY was identified by the following steps: splenic T cells derived from immunocompetent rats immunized with HTLV-I-infected cell lines were stimulated with formalin-fixed HTLV-I-infected cell lines; HTLV-I-specific CTL cell lines were established; cytotoxic activities of the above-mentioned HTLV-I-specific CTL cell lines against target cells G14 sensitized with synthetic peptides which are candidate for an epitope were measured. By immunizing immunocompetent rats with said epitope synthetic peptide and an adjuvant, then inducing tumor antigen epitope-specific CTLs, the proliferation of HTLV-I-infected tumor cells in vivo can be suppressed.

FIG 1

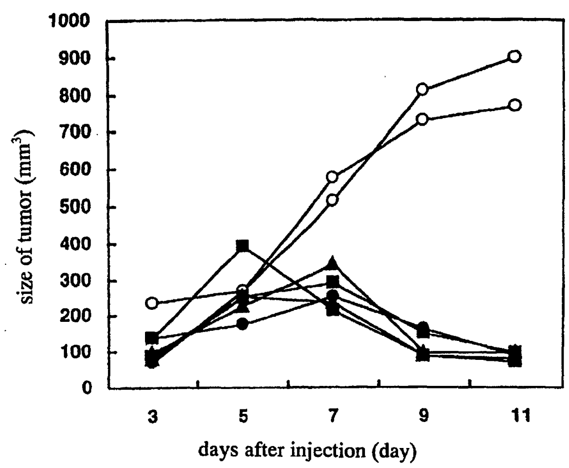


EP 1 391 729 A1

SEQUENCE LISTING

<110> JAPAN SCIENSE AND TECHNOLOGY CORPORATION

<120> Anti-tumor antigens or their epitopes against HTLV-I tumor

<130> A031-38PCT

<140>
<141>

<150>JP P2001-137526
<151>2001-05-08

<160> 6

<170> PatentIn Ver. 2.1

<210> 1
<211> 353
<212> PRT
<213> Human T-cell lymphotropic virus type 1

<400> 1
Met Ala His Phe Pro Gly Phe Gly Gln Ser Leu Leu Phe Gly Tyr Pro
1 5 10 15

Val Tyr Val Phe Gly Asp Cys Val Gln Gly Asp Trp Cys Pro Ile Ser
20 25 30

Gly Gly Leu Cys Ser Ala Arg Leu His Arg His Ala Leu Leu Ala Thr
35 40 45

Cys Pro Glu His Gln Ile Thr Trp Asp Pro Ile Asp Gly Arg Val Ile
50 55 60

Gly Ser Ala Leu Gln Phe Leu Ile Pro Arg Leu Pro Ser Phe Pro Thr

65 70 75 80

Gln Arg Thr Ser Lys Thr Leu Lys Val Leu Thr Pro Pro Ile Thr His
85 90 95

Thr Thr Pro Asn Ile Pro Pro Ser Phe Leu Gln Ala Met Arg Lys Tyr
100 105 110

Ser Pro Phe Arg Asn Gly Tyr Met Glu Pro Thr Leu Gly Gln His Leu
115 120 125

Pro Thr Leu Ser Phe Pro Asp Pro Gly Leu Arg Pro Gln Asn Leu Tyr
130 135 140

Thr Leu Trp Gly Gly Ser Val Val Cys Met Tyr Leu Tyr Gln Leu Ser
145 150 155 160

Pro Pro Ile Thr Trp Pro Leu Leu Pro His Val Ile Phe Cys His Pro
165 170 175

Gly Gln Leu Gly Ala Phe Leu Thr Asn Val Pro Tyr Lys Arg Ile Glu
180 185 190

Glu Leu Leu Tyr Lys Ile Ser Leu Thr Thr Gly Ala Leu Ile Ile Leu
195 200 205

Pro Glu Asp Cys Leu Pro Thr Thr Leu Phe Gln Pro Ala Arg Ala Pro
210 215 220

Val Thr Leu Thr Ala Trp Gln Asn Gly Leu Leu Pro Phe His Ser Thr
225 230 235 240

Leu Thr Thr Pro Gly Leu Ile Trp Thr Phe Thr Asp Gly Thr Pro Met
245 250 255

Ile Ser Gly Pro Cys Pro Lys Asp Gly Gln Pro Ser Leu Val Leu Gln
260 265 270

Ser Ser Ser Phe Ile Phe His Lys Phe Gln Thr Lys Ala Tyr His Pro
275 280 285

Ser Phe Leu Leu Ser His Gly Leu Ile Gln Tyr Ser Ser Phe His Ser
290 295 300

Leu His Leu Leu Phe Glu Glu Tyr Thr Asn Ile Pro Ile Ser Leu Leu
305 310 315 320

Phe Asn Glu Lys Glu Ala Asp Asp Asn Asp His Glu Pro Gln Ile Ser
325 330 335

Pro Gly Gly Leu Glu Pro Pro Ser Glu Lys His Phe Arg Glu Thr Glu
340 345 350

Val

<210> 2
<211> 9
<212> PRT
<213> Human T-cell lymphotropic virus type 1

<400> 2
Gly Ala Phe Leu Thr Asn Val Pro Tyr
1 5

<210> 3
<211> 9
<212> PRT
<213> Human T-cell lymphotropic virus type 1

<400> 3
Ala Phe Leu Thr Asn Val Pro Tyr Lys
1 5

<210> 4
<211> 9
<212> PRT
<213> Human T-cell lymphotropic virus type 1

<400> 4
Phe Leu Thr Asn Val Pro Tyr Lys Arg
1 5

<210> 5
<211> 22
<212> DNA
<213> Artificial Sequence

<220>
<223> Description of Artificial Sequence:ISS-ODN

<400> 5
tgactgtgaa cgttcgagat ga 22

<210> 6
<211> 9
<212> PRT
<213> Influenza A virus

<400> 6
Gly Ile Leu Gly Phe Val Phe Thr Leu
1 5

## Description

### Technical Field

The present invention relates to: a screening method for a CTL recognition antigen or an antigen epitope thereof which can induce cytotoxic T lymphocytes (CTLs) having an anti-tumor effect against human T cell leukemia virus type I (HTLV-I) tumors such as adult T cell leukemia (ATL), etc.; a screening method for an adjuvant that enhances the inducing activity of CTLs having an anti-tumor effect against HTLV-I tumors by a CTL recognition antigen or an antigen epitope thereof or an antigen epitope peptide thereof which can induce CTLs having an anti-tumor effect against HTLV-I tumors; a vaccine and the like for inducing an immune response containing said CTL recognition antigen or antigen epitope thereof, or DNA thereof as an active ingredient.

### Background Art

HTLV-I is involved in the pathogenesis of ATL, HTLV-I-associated myelopathy/tropical spastic paraparesis (HAM/TSP), as well as other inflammatory diseases (Blood 50, 481-492, 1977; Proc. Natl. Acad. Sci. USA 77, 7415-7419, 1980; Proc. Natl. Acad. Sci. USA 78, 6476-6480, 1981; Lancet. 2, 407-410, 1985; Lancet. 1, 1031-1032, 1986). HTLV-I is considered to have a specific sequence called pX between its env and 3'LTR, wherein this pX region cannot be seen in carcinogenic retroviruses of other animals, and its production called Tax protein is considered to play an important role for various pathogenesis of HTLV-I. It is also known that said HTLV-I Tax is a virus control protein and can immortalize rat and human cells in vitro (Proc. Natl. Acad. Sci. USA 87, 1071-1075, 1990; Blood 86, 4243-4249, 1995; J. Virol. 66, 4570-4575, 1992). These findings strongly indicate that HTLV-I Tax is involved in the mechanisms of HTLV-I-induced leukemogenesis. However, the mechanisms of pathogenesis of ATL in vivo are still unclear.

A number of studies have revealed that the levels of host cellular immunity against HTLV-I in ATL patients differ from those in HAM/TSP patients, and that HTLV-I-specific CTLs can be found in HAM/TSP patients and asymptomatic HTLV-I carriers but CTLS are only rarely found in ATL patients (J. Immunol. 130, 2942-2946, 1983; J. Exp. Med. 158, 994-999, 1983; J. Immunol. 133, 1037-1041, 1984; Nature 348, 245-248, 1990; Virology 188, 628-636, 1992; Int. J. Cancer 54, 582-588, 1993). Based on these observations, it is considered that host cellular immunity may influence pathogenesis of HTLV-I-associated diseases.

In general, the above-mentioned CTLs play an important role not only in viral clearance, but also in tumor eradication. It is reported by the present inventors and other groups that HTLV-I-specific $CD8^+$ CTLs in HTLV-I carriers recognize HTLV-I Tax (Nature 348, 245-248, 1990; Int. Immunol. 3, 761-767, 1991), and lyse ATL cells in vitro (J. Immunol. 133, 1037-1041, 1984; Int. J. Cancer 54, 582-588, 1993). These observations indicate that HTLV-I-specific CTLs may be an important effector of host immunosurveillance against HTLV-I-induced tumor proliferation. However, it is still unclear whether the above-mentioned role of CTLs is against HTLV-I tumors in vivo or merely a consequence of infection.

To clarify the effect of CTLs on HTLV-I leukemogenesis in vivo, the present inventors developed two experimental rat model systems of ATL-like diseases (J. Virol. 73, 6031-6040, 1999; J. Virol. 74, 428-435, 2000; Japanese Patent Application No.H10-315174). One is a model of T cell lymphomas in athymic rats following inoculation of HTLV-I-immortalized rat cell lines (J. Virol. 73, 6031-6040, 1999). In this model, adoptive transfer of immune T cells protected rats from fatal lymphomas. In the other model, the development of T cell lymphomas was induced by treatment with anti-CD80 and -CD86 monoclonal antibodies which block costimulatory signals for T cell activation in immunocompetent rats (J. Virol. 74, 428-435, 2000). These findings strongly indicate that the role of a host T cell immune response as to prevent the proliferation of HTLV-I tumors in vivo.

The observations made in studies of CTLs in human HTLV-I-infected patients and the above-mentioned rat models indicate that augmentation of HTLV-I-specific CTLs in pre-ATL patients might protect them from pathogenesis of ATL. Since the recovery rate of ATL is extremely low among lymphoproliferative disorders because of its resistance to chemotherapy, the development of therapies by immunological approach at an early stage of diseases have been expected. In order to induce an effective anti-tumor immune response, the tumor antigen must be precisely clarified and a strong immunogen recognized by cellular immunity must be administered to the host. In particular, to induce a $CD8^+$ CTL response which is one of the major populations that recognize tumors, presentation of an appropriate peptide processed by MHC class I as well as class II antigens of antigen-presenting cells is essential (Nature 343, 692-696; 1989). Because of the preference of the pathway of antigen presentation by MHC class I, a variety of viral vectors have been proposed for delivery and expression of exogenous genes that encode target antigens (J. Natl. Cancer Inst. 90, 1894-1900, 1998). It is considered that similar effects can be obtained by the direct injection of untreated DNA (Annu. Rev. Immunol. 18, 927-974, 2000). In addition, peptide-based vaccines corresponding to CTL epitopes that directly bind to MHC molecules, which are safer than DNA-based vaccines, are also under consideration (Int. J. Cancer 63, 883-885, 1995).

ATL is a neoplastic disease caused by the infection of HTLV-I which shows high carrier rate in Japan, however, it has been considered to be a malignant tumor with extremely bad prognosis because of its resistance to chemotherapeutic agents. On the other hand, various clinical observations indicate the anti-tumor effect of host cellular immunity, particularly of CTLs. The object of the present invention is to provide: a screening method for a CTL recognition antigen or an antigen epitope thereof which can induce CTLs having an anti-tumor effect against HTLV-I tumors such as ATL, etc.; a screening method for an adjuvant that enhances the inducing activity of CTLs having an anti-tumor effect against HTLV-I tumors by a CTL recognition antigen or an antigen epitope thereof or an antigen epitope peptide thereof which can induce CTLs having an anti-tumor effect against HTLV-I tumors; a vaccine and the like for inducing an immune response containing said CTL recognition antigen or antigen epitope thereof, or DNA thereof as an active ingredient.

**Disclosure of the Invention**

The present inventors made a keen study to attain the object mentioned above. Splenic T cells derived from syngeneic immunocompetent rat immunized with nu/+ rat-derived HTLV-I-infected cell lines, FPM1-V1AX, were repeatedly stimulated every 2 weeks by using formalin-fixed FPM1-V1AX to establish HTLV-I-specific CTL cell lines, and the cytotoxic activity of the above-mentioned HTLV-I-specific CTL cell lines against target cells G14 sensitized with various synthetic peptides that are considered to include the dominant recognition epitope of HTLV-1-specific CTLs were studied, and the present inventors identified a peptide for which target cells show particularly strong CTL sensitivity, namely the dominant epitope of a CTL recognition antigen which can induce CTLs having an anti-tumor effect against HTLV-I tumors. The present inventors verified that it is possible to induce tumor antigen epitope-specific CTLs in immunocompetent rats by using synthetic peptides of said epitope as an immunogen and using an adjuvant, and that said induced CTLs can strongly suppress proliferation of HTLV-I-infected tumor cells in vivo. Thus, the present invention has completed.

The present invention relates to: a screening method for a CTL recognition antigen or an antigen epitope thereof which can induce cytotoxic T lymphocytes (CTLs) having an anti-tumor effect against HTLV-I tumors, wherein CTLs induced by a test substance is administered to a non-human animal model of HTLV-I-associated disease, and the change of tumors in said non-human animal is measured and assessed (claim 1); the screening method for a CTL recognition antigen or an antigen epitope thereof which can induce CTLs having an anti-tumor effect against HTLV-I tumors according to claim 1, wherein the non-human animal model of HTLV-I-associated disease is a non-human animal model of adult T cell leukemia (claim 2); the screening method for a CTL recognition antigen or an antigen epitope thereof which can induce CTLs having an anti-tumor effect against HTLV-I tumors according to claim 1 or 2, wherein the non-human animal is a rat (claim 3); a screening method for a CTL recognition antigen or an antigen epitope thereof which can induce CTLs having an anti-tumor effect against HTLV-I tumors, wherein CTLs induced by a test substance is contacted with HTLV-I-infected tumor cell lines, and the cytotoxic activity of said CTLs is measured and assessed (claim 4); a screening method for a CTL recognition antigen or an antigen epitope thereof which can induce CTLs having an anti-tumor effect against HTLV-I tumors, wherein target cells sensitized with a test substance or target cells that express a test substance are contacted with HTLV-I-specific CTL cell lines, and the cytotoxic activity of said HTLV-I-specific CTL cell lines is measured and assessed (Claim 5); the CTL recognition antigen or the antigen epitope thereof which can induce CTLs having an anti-tumor effect against HTLV-I tumors, which is obtained by the screening method according to any of claims 1 to 5 (claim 6); the antigen epitope according to claim 6, wherein the antigen epitope is a peptide comprising an amino acid sequence shown by Seq. ID No.2 (claim 7).

The present invention further relates to: a screening method for an adjuvant that enhances an anti-tumor effect against HTLV-I tumors, wherein CTLs induced by using the CTL recognition antigen or the antigen epitope thereof according to claim 6 and a test adjuvant are administered to a non-human animal model of HTLV-I-associated disease and the change of tumors in said non-human animals is measured and assessed (claim 8); the screening method for an adjuvant that enhances an anti-tumor effect against HTLV-I tumors according to claim 8, wherein the non-human animal model of HTLV-I-associated disease is a non-human animal model of adult T cell leukemia (claim 9); the screening method for an adjuvant that enhances an anti-tumor effect against HTLV-I tumor according to claim 8 or 9, wherein the non-human animal is a rat (claim 10); a screening method for an adjuvant that enhances an anti-tumor effect against HTLV-I tumors, wherein CTLs induced by using the CTL recognition antigen or the antigen epitope thereof according to claim 6 and a test adjuvant are contacted with HTLV-I-infected tumor cell lines, and the cytotoxic activity of said CTLs is measured and assessed (claim 11); the screening method for an adjuvant that enhances an anti-tumor effect against HTLV-I tumors according to any of claims 8 to 11, wherein the antigen epitope peptide according to claim 7 is used as the CTL recognition antigen or the antigen epitope thereof according to claim 6 (claim 12).

The present invention still further relates to: a vaccine for inducing an immune response containing a CTL recognition antigen or an antigen epitope thereof which can induce CTLs having an anti-tumor effect against HTLV-I tumors and which can be obtained by the screening method according to any of claims 1 to 5 as an active ingredient (claim 13); a vaccine for inducing an immune response containing a DNA that encodes a CTL recognition antigen or an antigen epitope thereof which can induce CTLs having an anti-tumor effect against HTLV-I tumors obtained by the screening method according to any of claims 1 to 5 as an active ingredient (claim 14); the vaccine for inducing an immune response according to claim 13 or 14, wherein the CTL recognition antigen is an HTLV-I Tax protein shown by Seq. ID No.1 (claim 15); the vaccine for inducing an immune response according to claim 13 or 14, wherein the CTL recognition antigen is a protein comprising an amino acid sequence wherein one or a few amino acids are deleted, substituted, or added in the amino acid sequence shown by Seq. ID No. 1, which can induce CTLs having an anti-tumor action against HTLV-I tumors (claim 16); the vaccine for inducing an immune response according to claim 13 or 14, wherein the CTL recognition antigen epitope is a peptide comprising the amino acid sequence shown by Seq. ID No.2 (claim 17); the vaccine for inducing an immune response according to claim 13 or 14, wherein the CTL recognition antigen epitope is a peptide comprising an amino acid sequence wherein one or a few amino acids are deleted, substituted, or added in the amino acid sequence shown by Seq. ID No.2, which can induce the CTLs having an anti-tumor action against HTLV-I tumors (claim 18); the vaccine for inducing an immune response according to any of claims 13 to 18, further containing an adjuvant that enhances an anti-tumor effect against HTLV-I tumors (claim 19); the vaccine for inducing an immune response according to claim 19, wherein the adjuvant which can be obtained by the screening method according to any of claims 8 to 12 is an adjuvant that enhances an anti-tumor effect against HTLV-I tumors (claim 20); the vaccine for inducing an immune response according to claim 20, wherein the adjuvant that enhances an anti-tumor effect against HTLV-I tumors which can be obtained by the screening method according to any of claims 8 to 12 is ISS-ODN (claim 21); an HTLV-I recognition CTL induced by the vaccine for inducing an immune response according to any of claims 13 to 21 (claim 22); a pharmaceutical composition containing the HTLV-I recognition CTL according to claim 22 as an active ingredient (claim 23).

**Brief Description of Drawings**

Fig. 1 is a figure showing the results of examining a subset of immune T cells considered to be directly required for the regression of tumors in vivo.
Fig. 2 is a figure showing the results of cytotoxic activities against target cells of various splenic T cells derived from rats wherein tumors have been completely regressed.
Fig. 3 is a figure showing the results of examining viral antigens recognized by HTLV-I-specific CTLs.
Fig. 4 is a figure showing the results of inhibition of cytotoxicity against target cells of HTLV-I-specific CTL cell lines in the presence of a competitor.
Fig. 5 is a figure showing the results of cytotoxic activities of various CTLs induced by HTLV-I-infected tumor cell line FPM1-V1AX.
Fig. 6 is a figure showing the results of screening of HTLV-I-specific CTL cell line recognition peptides.
Fig. 7 is a figure showing the results of screening of HTLV-I-specific CTL cell line recognition peptides in the case where long-term-cultured CTL cell lines are used.
Fig. 8 is a figure showing the results of screening 9 amino acid synthetic peptides recognized by HTLV-I-specific CTL cell lines.
Fig. 9 is a figure showing the results of examining the cytotoxic activity of CTLs against target cells sensitized with various concentrations of peptides.
Fig. 10 is a figure showing the specificity of HTLV-I-specific CTL cell lines against synthetic peptide Tax 180-188.
Fig. 11 is a figure showing the results of proliferation suppression effect on HTLV-I tumors in vivo by Tax 180-188 recognition CTL cell lines.
Fig. 12 is a figure showing the effectiveness of Tax 180-188 and ISS-ODN as vaccines.

**Best Mode of Carrying Out the Invention**

The screening method for a CTL recognition antigen or an antigen epitope thereof which can induce CTLs having an anti-tumor effect against HTLV-I tumors of the present invention can be particularly exemplified by the following: a method wherein CTLs induced by a test substance are administered to a non-human animal model of HTLV-I-associated disease such as ATL and the like, and the change of tumors in said non-human animal is measured and assessed; a method wherein CTLs induced by a test substance is contacted with HTLV-I-infected tumor cell lines, and cytotoxic activity of said CTLs is measured and assessed; a method wherein target cells sensitized with a test substance or target cells expressing a test substance is contacted with HTLV-I-specific CTL cell lines, and the cytotoxic activity of said HTLV-I-specific CTL cell lines is measured and assessed. As used herein, the CTL recognition antigen or the antigen epitope thereof which can induce CTLs means a CTL recognition antigen or an antigen epitope thereof which can induce CTLs in vivo and in vitro.

The above-mentioned non-human animal model of HTLV-I-associated disease is not particularly restricted as long as it is a non-human animal which induces HTLV-I-associated diseases such as ATL, HAM/TSP, HAAP, uveitis, alveobronchiolitis, sialadenitis akin to Sjögren syndrome, etc., by infection of HTLV-I. However, the one that can reproducibly proliferate HTLV-I-infected tumor cells over long-term period is preferable. Besides, the non-human animal of the present invention can be particularly exemplified by non-human mammals such as mice, rats, guinea pigs, monkeys, cats or rabbits, but they are not limited to these examples. The method of generating a non-human animal model of ATL will be explained below with an ATL rat model as an example.

An ATL rat model can be obtained by, for instance, administering HTLV-I-infected tumor cell lines to an immunocompetent rat. However, an ATL rat model which can be obtained by administering HTLV-I-infected tumor cell lines subcutaneously, intraperitoneally, intravenously or the like to a non-human animal which is deficient in T cell function is more preferable in view of the reproducibility, and the point that HTLV-I-infected tumor cells can be proliferated and subcultured in vivo. As for a wild type rat used for evaluation, it is preferable to use a wild type rat syngeneic to an ATL rat model. The above-mentioned non-human animal which is deficient in T cell function can be particularly exemplified by a nude non-human animal and the like, but they are not limited to these examples. In addition, there is no limitation to the above-mentioned HTLV-I-infected tumor cell lines as long as they are infected with HTLV-I by a known method and of which MHCs coincide with a wild type non-human animal. For instance, it can be particularly exemplified by cell lines such as FPM1-V1AX and the like.

The test substance used for the screening methods of the present invention can be particularly exemplified by proteins, peptides, DNAs, RNAs, antisense DNAs, antisense RNAs, etc. There is no limitation to the target cells sensitized with the above-mentioned test substance or the target cells that express a test substance as long as the MHCs thereof coincide, however, it can be preferably exemplified by G14 cells, which are $CD8^+$ T cell lines (J. Virol. 74, 9610-9616, 2000).

There is no limitation to the expression system for preparation of cells that express a test substance as long as the expression system is capable of expressing the above-mentioned test substance intracellularly and the examples include, chromosome-, episome-, and virus-derived expression systems, for instance, vectors derived from bacterial plasmid, vectors derived from yeast plasmid, vectors derived from papovavirus such as SV40, vaccinia virus, adenovirus, fowlpox virus, pseudorabies virus, retrovirus, and vectors derived from bacteriophage or transposon and vectors derived from the combination of these two, e.g. vectors derived from genetic factors of plasmid and bacteriophage, such as cosmid and phagemid. This expression system may contain a regulatory sequence that not only induces expression but also regulate the expression. In addition, an expression vector series which is capable of translating by changing the reading frame can also be used advantageously.

The expression system wherein said test substance is incorporated can be introduced into a host cell by the methods described in many standard laboratory manuals such as manuals of Davis et al. (BASIC METHODS IN MOLECULAR BIOLOGY, 1986) and of Sambrook et al. (MOLECULAR CLONING: A LABORATORY MANUAL, 2nd Ed., Cold Spring Harbor Laboratory Press, Cold Spring Harbor, N.Y., 1989), and can be conducted by the examples including calcium phosphate transfection, DEAE-dextran-mediated transfection, transvection, microinjection, cationic lipid-mediated transfection, electroporation, transduction, scrape loading, ballistic introduction, infection, etc.

There is no particular limitation to the HTLV-I-specific CTL cell lines of the present invention as long as they are the cell lines that specifically recognize HTLV-I, however, the cell lines restrained by MHC class I are more preferable. Said HTLV-I-specific CTL cell lines restrained by MHC class I can be obtained by a known method, for instance, a method wherein non-human animal-derived T cells that express MHC class I molecules are infected with HTLV-I, and proliferated in the body of a non-human animal which is deficient in T cell function (nude non-human animal), to establish HTLV-I-infected tumor cell lines, then the splenic T cells of the immunocompetent non-human animal which is immunized with such established cell lines are repeatedly stimulated with formalin-fixed HTLV-I-infected tumor cell lines.

The CTL recognition antigen which can be obtained by the above-mentioned screening method and which is capable of inducing CTLs having an anti-tumor effect against HTLV-I tumors can be exemplified by an HTLV-I Tax protein shown by Seq. ID No.1, a protein comprising an amino acid sequence wherein one or a few amino acids are deleted, substituted, or added in the amino acid sequence shown by Seq. ID No.1 and which is capable of inducing CTLs having an anti-tumor action against HTLV-I tumors or the like. The antigen epitope which can be obtained by the above-mentioned screening method can be particularly exemplified by a peptide comprising an amino acid sequence shown by Seq. ID Nos. 2, 3, or 4, or particularly preferably, a peptide comprising an amino acid sequence shown by Seq. ID No.2, a peptide comprising an amino acid sequence wherein one or a few amino acids are deleted, substituted, or added in these amino acid sequences and which is capable of inducing CTLs having an anti-tumor action against HTLV-I tumors or the like, however, such antigen epitopes are not limited to these examples.

The screening method for an adjuvant that enhances an anti-tumor effect against HTLV-I tumors of the present invention, namely, an adjuvant which is capable of inducing CTLs specific to a CTL recognition antigen or an antigen epitope thereof having a more efficient anti-tumor effect against HTLV-I tumors is not particularly restricted as long as such method includes: a method wherein CTLs induced by using the above-mentioned CTL recognition antigen or an antigen epitope thereof, or preferably, an antigen epitope peptide comprising the amino acid sequence shown by Seq. ID No.2 and a test adjuvant are administered to a non-human animal model of HTLV-I-associated disease such as adult T cell leukemia non-human animal model and the like, and the change of tumors in said non-human animal is measured and assessed; a method wherein CTLs induced by using the above-mentioned CTL recognition antigen or the antigen epitope thereof, or preferably, CTLs induced by using an antigen epitope peptide comprising the amino acid sequence shown by Seq. ID No.2 and a test adjuvant is contacted with HTLV-I-infected tumor cell lines, and cytotoxic activity of said CTLs is measured and assessed. The adjuvant which can be obtained by such screening method can be particularly exemplified by the following examples: ISS-ODN that includes CpG motif which is capable of efficiently inducing peptide-specific CTLs (Immunostimulatory DNA sequences-oligodeoxynucleotide; Nat. Med. 3, 849-854, 1997), QS21 that stimulates cytotoxic T cells (Quillaia saponaria; which can be commercially obtained from Cambridge Biotech, Worcester, MA), aluminum hydroxide, aluminum phosphate, aluminum oxide, oil-based emulsion, saponin, vitamin E lysate, etc.

The CTL recognition antigen or the antigen epitope thereof, or DNA encoding them which can be obtained by the aforementioned screening method and which can induce CTLs having an anti-tumor effect against HTLV-I tumors can be used as a vaccine for inducing an immune response such as cellular immuniny, humoral immunity and the like. The vaccine for inducing an immune response of the present invention can be exemplified by a CTL recognition antigen which can induce CTLs having an anti-tumor effect against HTLV-I tumors, of which examples include: an HTLV-I Tax protein shown by Seq. ID No.1; a protein comprising an amino acid sequence wherein one or a few amino acids are deleted, substituted, or added in the amino acid sequence shown by Seq. ID No.1 and which can induce CTLs having an anti-tumor action against HTLV-I tumors, or a CTL recognition antigen epitope, of which examples include: a vaccine for inducing an immune response containing a peptide comprising the amino acid sequence shown by Seq. ID No.2, or a peptide comprising an amino acid sequence wherein one or a few amino acids are deleted, substituted, or added in the amino acid sequence shown by Seq. ID No. 2, and which can induce CTLs having an anti-tumor action against HTLV-I tumors as an active ingredient, or a vaccine for inducing an immune response containing DNA that encodes the above-mentioned CTL recognition antigen and the antigen epitope thereof which can induce CTLs having an anti-tumor action against HTLV-I tumors as an active ingredient. As for the vaccine for inducing an immune response of the present invention, the one containing various adjuvants that further enhances cellular or local immunity is preferable. Said adjuvant can be exemplified by the one can be obtained by a screening method for an adjuvant that enhances an anti-tumor effect against the aforementioned HTLV-I tumors such as ISS-ODN and the like. When an adjuvant is used, it can also be used as a recombinant fusion protein or a recombinant fusion peptide generated from DNA which continuously encodes various fungus components, toxins and the like to be used as an adjuvant together with the above-mentioned CTL recognition antigen or an antigen epitope thereof, preferably an antigen epitope peptide comprising the amino acid sequence shown by Seq. ID No.2.

The vaccine for inducing an immune response of the present invention may contain a pharmaceutically acceptable carrier or a diluent, an immunostimulator, an additive agent, etc. The carrier or diluent can be particularly exemplified by the following: a stabilizing agent such as SPGA; carbohydrates such as sorbitol, mannitol, starch, sucrose, glucose, dextran and the like; proteins such as albumin, casein and the like; protein-containing substances such as bovine sera, skim milk and the like; buffer solutions such as phosphate buffer solution, physiological saline, water and the like. The immunostimulator can be particularly exemplified by cytokines such as interleukin-2 (IL-2), interleukin-12 (IL-12), tumor necrosis factor $\alpha$ (TNF-$\alpha$) and the like. The additive agent can be exemplified by polypeptides of low molecular weight (less than approximately 10 residues), proteins, amino acids, carbohydrate containing glucose or dextran, chelating agents such as EDTA and the like, protein stabilizing agents, inhibitors or suppressors of microorganism proliferation and the like, but they are not limited to these examples.

The pharmaceutical agents or pharmaceutical compositions provided by the present invention can be exemplified by the one containing HTLV-I recognition CTLs induced by administering a vaccine for inducing an immune response to non-human animals and the like as an active ingredient, as well as the above-mentioned vaccine for inducing an immune response. Preferable forms for these pharmaceutical agents or pharmaceutical composition are the forms which can be administered orally, intravenously, intraperitoneally, intranasally, intracutaneously, subcutaneously, intramascularly and the like. Effective doses to be administered can be determined accordingly by considering the types and compositions of such pharmaceutical agents or pharmaceutical compositions, the administration methods, the age and body weight of a patient, etc. It is preferable to administer them one or a few times a day. When administered orally, it is ordinarily administered through the formulations prepared by mixing with a carrier for formulation. Here, a substance which is usually used in the formulation field, and which does not react with the peptide of the present invention is used as a carrier for formulation.

Further, dosage forms can be particularly exemplified by tablets, capsules, granules, powder, syrup, suspension agent, suppository, ointment, cream, gel, patch, inhalant, injectable solution, etc. These formulations are prepared by ordinary protocols, and particularly, liquid formulation can be dissolved or suspended into water or other appropriate media when used. Tablets and granules may be coated by a known method. Injectable solution is prepared by dissolving the peptide of the present invention into water, however, it may also be dissolved into physiological saline or glucose solution according to need, and buffer agent or preservation agent may be further added. These formulations may also contain other ingredients of therapeutic value.

The CTL recognition antigen or the antigen epitope thereof which can induce CTLs having an anti-tumor effect against HTLV-I tumors of the present invention can also be ingested as a functional food by compounding them into the following as food materials for infection control of HTLV-I and/or symptom improvement of HTLV-I-associated disease: baked goods such as puddings, cookies, breads, cakes, jellies, rice crackers, etc.; Japanese confectioneries such as sweet jellied adzuki-bean pastes, etc.; breads and confectioneries such as frozen desserts, chewing gums, etc; noodles such as Japanese wheat noodles, buckwheat noodles, etc.; fish paste products such as steamed fish pastes, hams, fish meat sausages; various beverages such as yogurts, yogurt drinks, juice, cow milk, soy milk, alcoholic beverages, coffee, tea, natural leaf tea, oolong tea, isotonic drinks, etc.; condiments such as soybean pastes, soy sauce, dressing, mayonnaise, edulcorant, etc.; bean-curds; devil's tongue; various prepared food such as fish boiled in soy sauce, dumplings, croquettes, salads, etc.

The present invention will be further particularly explained hereinafter with reference to the examples, but the scope of the invention will not be limited to these examples.

Example 1 (Cell Lines)

FPM1-V1AX (J. Virol. 73, 6031-6040, 1999) was established by proliferating immortalized rat T cell lines FPM1 established through the infection of HTLV-I with T cells of immunocompetent rats F344/N Jcl-rnu/+ (nu/+) (4-week-old female rats; Clea Japan, Inc.), in the body of a nude rat F344/N Jcl-rnu/rnu (nu/nu) (J. Virol. 73, 6436-6443, 1999). FPM-SV was established by transforming HTLV-I negative SV40 to nu/+ rat-derived renal cell lines (J. Virol. 73, 6031-6040, 1999). An HTLV-I-infected T cell line established from splenocytes of WKAH rats (TARS-1; J. Exp. Med. 159, 1105-1116, 1984) was kindly provided by Dr. T. Yoshiki of Hokkaido University and used in the experiment. $RT1.A^1$/TARS-1 was established by transfecting $RT1.A^1$-expressing plasmid pRep10 into TARS-1 cells followed by in vitro selection with 400 μg/ml of hygromycin. Besides, pRep10 (Immunogenetics 39, 447, 1994) was kindly provided by Dr. S. Salgar (Miami Univ. , FL) and used herein. The expression of $RT1.A^1$ in the above-mentioned $RT1.A^1$/TARS-1 cells was confirmed by immunofluorescence analysis. G14-Tax cell lines which are the cells that stably express G14 and Tax genes, i.e. the IL-2-dependent HTLV-I negative $CD8^+$ T cell lines established from nu/+ rats were prepared by the method as described previously (J. Virol. 74, 9610-9616, 2000). All the cell lines used herein were maintained in RPMI1640 containing 10% heat-inactivated FCS (Whittaker, Walkersville, MD), penicillin, and streptomycin. 10U/ml of recombinant human IL-2 (rhIL-2) (Shionogi Pharmaceutical & Co., Ltd.) was added to the culture medium RPMI1640 for maintaining G14 and G14-Tax.

Example 2 (Preparation of Immune T cell Subset)

4-week-old nu/+ rats were immunized with intraperitoneal administration of 2 x $10^7$ of FPM1-V1AX cells two times with a two-week interval. One week after the last immunization, splenic T cells were isolated and purified through a nylon wool column, then lymphocytes subsets were further purified using complement lysis method. To explain briefly, supernatants of splenic T cells were mixed with anti-rat CD8 monoclonal antibodies (R1-10B5) or anti-rat CD4 monoclonal antibodies (RTH-7), and incubated on ice for 30 minutes. After washed with 1% FCS-PBS, the above-mentioned cells were incubated in the culture medium RPMI1640 containing 2% rabbit serum (Cedarlane Laboratories Limited, Ontario, Canada) at 37° C for 45 minutes, then washed two times with 10% FCS-RPMI 1640. Subsequently, it was confirmed by a flow cytometry analysis whether $CD8^+$ or $CD4^+$ T cells were condensed to on and above 98%.

Example 3 (Anti-Tumor Effect of $CD4^+$ or $CD8^+$ T cells)

The present inventors previously reported that adoptive transfer of splenic T cells from rats immunized with HTLV-I-infected cells effectively inhibited the proliferation of malignant lymphomas in F344/N Jcl-rnu/rnu (nu/nu) rats wherein an HTLV-I-infected tumor cell line FPM1-V1AX was administered (J. Virol. 73, 6031-6040, 1999). Therefore, in order to clarify which subsets of immune T cells are directly required for tumor regression in vivo, anti-tumor effect against each subset of F344/N Jcl-rnu/rnu (nu/nu) rats was examined by using $CD4^+$ or $CD8^+$ cell groups isolated by the method described in Example 2. 2 x $10^7$ of FPM1-V1AX cell lines were subcutaneously inoculated into 4-week-old nu/nu female rats (Clea Japan, Inc.) that were simultaneously administered intraperitoneally with $10^7$ of $CD4^+$ T cells (▲), $CD8^+$ T cells (■), or total T cells (●) isolated in Example 2. Then suppressing effect on tumor proliferation was examined by measuring the size of each subcutaneous tumor with a caliper every other day. In these measurements, the longest surface length (mm;a) and width (mm;b) were determined by the above-mentioned measurement, and the

tumor volume (V;$mm^3$) was calculated using the formula ($V=ab^2/2$) as described previously (J. Virol. 73, 6031-6040, 1999). Besides, 4-week-old nu/nu rats subcutaneously inoculated with FPM1-V1AX cell lines alone served as controls (○).

The results of the above are shown in Fig. 1. It was confirmed that both $CD4^+$ and $CD8^+$ T cells exhibited equivalent inhibitory effect on the growth of HTLV-I-infected tumor cell lines as well as total T cells. No metastic tubercles were observed in the rats which underwent tranfer of any subsets of the immune T cells, in contrast to the visible tubercles in the lungs and mediastinal lymph nodes of tumor-bearing rats.

In order to conduct further analysis, splenic T cells ($CD4^+$ T cells, $CD8^+$ T cells, or total T cells) were isolated respectively from each nu/nu rat on day 28 when complete tumor regression occurred by transferring immune T cells ($CD4^+$ T cells, $CD8^+$ T cells, or total T cells), then their cytotoxic activities against the respective target cells were examined. The above-mentioned various splenic T cells ($5 \times 10^6$ cells/well) were cocultured with formalin-fixed FPM1-V1AX cells ($2 \times 10^6$ cells/well) in 2ml of 10% FCS-RPMI 1640 per well of a 24-well plate for 6 days. Cytotoxic activities against target cells were measured by $^{51}Cr$ release assay for 6 hours as described previously (Immunology 14, 181-196, 1968) at the ratio of said cells (E; effector cells) and target cells (T; FPM1-V1AX, syngeneic T cell lines G14, or G14-Tax) (E/T) as 10, and calculated by the formula as described below. In addition, values (mean ± SD) were evaluated from three independent experiments.

Formula 1

$$\frac{[\text{experimental } ^{51}\text{Cr release - spontaneous } ^{51}\text{Cr release}]}{[\text{maximum } ^{51}\text{Cr release - spontaneous } ^{51}\text{Cr release}]} \times 100\%$$

The results of the above are shown in Fig. 2. From these results, various T cells showed high cytotoxic activities against FPM1-V1AX and G14-Tax, however, they did not show a cytotoxic activity against HTLV-I negative G14 cells. In addition, it was notably recognized by flow cytometry that the splenic T cells isolated from nu/nu rats wherein the above-mentioned various immune T cells ($CD4^+$ or $CD8^+$ immune T cells) were transferred, were positive for CD4 or CD8 respectively. These results indicate that an HTLV-I-specific cytotoxic activity of $CD4^+$ or $CD8^+$ T cells is strongly involved in direct elimination of HTLV-I-infected tumor cells in vivo.

Example 4 (Recombinant Vaccinia Virus)

Subsequently, in order to examine viral antigens recognized by HTLV-I-specific CTLs, the following recombinant vaccinia viruses containing 4 types of HTLV-I genes provided by Dr. H. Shida, Hokkaido University (rvv; Embo J. 6, 3379-3384, 1987; J. Virol. 62, 3718-3728, 1988; Cell 55, 197-209, 1988) were used herein: rvv containing HTLV-I env gene (WR-env); rvv containing HTLV-I gag gene (WR-gag); rvv containing HTLV-I pX gene (WR-40X and WR-27X). WR-27X is a recombinant vaccinia virus that expresses p21X, p27rex, and p40tax, whereas WR-40X is the one that expresses p21X and p40tax. Each of the above-mentioned HTLV-I-rvv (WR-40X, WR-27X, WR-env, WR-gag) was infected with FPM-SV cells at a m.o.i (multiplicity of infection) of 10 at 37° C for 1 hour. After incubation, the cells were washed once with 10% FCS-RPMI 1640, then cultured in 10% FCS-RPMI 1640 at 37°C for 12 hours. Said infected cells were labeled with a radioactive isotope in order to be used for the following example.

Example 5 (HTLV-I Antigen Recognized by Rat CTLs)

Splenic T cells isolated from nu/+ rats that had been intraperitoneally administered with FPM1-V1AX were stimulated with formalin-fixed FPM1-V1AX for 6 days, and then used as effector cells in the following example. As the target cells, FRM1-V1AX, FPM-SV, and various HTLV-I-rvv-infected FPM-SV cells (WR-40X, WR-27, WR-env, WR-gag) prepared in Example 4 were used. In addition, the cells wherein rvv which does not contain an HTLV-I gene are infected with FPM-SV cells (WR-HA) were used as controls. As a result of immunofluorescence analysis conducted for the above-mentioned HTLV-I-rvv-infected FRM-SV cells, these cells showed positive for MHC class I, but negative for MHC class II antigen. Therefore, these cells are considered to selectively express an antigen confined to MHC class I.

In the same way as described in Example 3, $^{51}Cr$ release assay was conducted using the above-mentioned effector cells (E) and target cells (T) at each E/T ratio as shown in Fig. 3. Measurements (mean ± SD) were evaluated from three independent experiments. The results are shown in Fig. 3. These results show that effector cells have high cytotoxicity against FPM1-V1AX, but not FPM-SV cells. Among HTLV-I-rvv-infected FPM-SV cells, WR-40X- and WR-27X-infected cells, which commonly express HTLV-I tax, showed the highest sensitivity for effector cells. The target cells that express HTLV-I env (envelope) also showed sensitivity, but to a lesser degree. However, cytotoxic activities against FPM-SV cells that express HTLV-I gag were as low as the cytotoxic activities against target cells (WR-HA) as controls.

Subsequently, the inhibition of cytotoxicity of HTLV-I-specific CTL cell lines against target cells by unlabeled cells was examined. The $CD8^+$ CTL cell lines which are more specific to HTLV-I Tax obtained by the long-term culture and the target cells ($[^3H]$-TdR-labeled FPM1-V1AX:5 x $10^3$ cells /well) were plated on 96-well U-bottom plates at an E/T ratio of 10, then a competitor [unlabeled FPM1-V1AX (●), G14-Tax (■), or G14 (▲)] was added at a competitor-to-target ratio as described in Fig.4, and incubated at 37°C for 6 hours. Thereafter, cells were harvested with a Micro 96 Harvester (Skatron), then the amount of remaining target cells was measured by a microplate β-counter (Micro Beta Plus), and the specific cytotoxicity (%) was calculated by the following formula. Values (mean ± SD) were evaluated from three independent experiments. The cells which were incubated with 3.7 MBq of $[^3H]$-TdR per $10^6$ cells at 37°C for 12 hours, then washed three times in advance were used as the above-mentioned target cells.

Formula 2

$$\frac{\text{[cpm without the presence of an effector - cpm in the presence of an effector]}}{\text{[cpm without the presence of an effector]}} \times 100\%$$

The results of the above are shown in Fig. 4. These results show that cytotoxic activities of CTL cell lines against FPM1-V1AX are notably inhibited by increasing unlabeled G14-Tax but not G14 cells. These results indicate that HTLV-I Tax is one of the major antigen specifically recognized by CTLs derived from nu/+ rats administered with syngeneic HTLV-I-infected cells in vivo.

Example 6 (MHC Class I Restrain of HTLV-I-Specific Cytotoxic Activity in nu/+ Rats)

For induction of HTLV-I-specific CTL cell lines (two types of $CD8^+$ CTL cell lines and $CD4^+$ CTL cell lines) in long-term culture, 2.5 x $10^6$ cells /well of splenic T cells were cocultured with the same number of formalin-fixed FPM1-V1AX cells in 10% FCS-RPMI 1640 wherein 20 U/ml of rhIL-2 was added, with periodical stimulation using formalin-fixed FPM1 cells every two weeks, and two types of $CD8^+$ CTL cell lines ($CD8^+$ CTL-1 and $CD8^+$ CTL-2) and $CD4^+$ CTL cell lines were established. Then, the MHC class I restrain against cytotoxic activities was examined in the same way as Example 5 using such induced HTLV-I-specific CTL cell lines. As target cells, 4 cell lines labeled with $[^3H]$-TdR in the same way as in Example 5 were used: nu/+ rat-derived FPM1-V1AX that expresses a rat MHC class I molecule, $RT1.A^1$ X; WKAH rat-derived W7KSV that does not express $RT1.A^1$; WKAH rat-derived TARS-1 that does not express $RT1.A^1$; a cell line $RT1.A^1$/TARS-1 established by stably transfecting $RT1.A^1$ cDNA to TARS-1 cells.

The results of the above are shown in Fig. 5. Two types of $CD8^+$ CTL cell lines ($CD8^+$ CTL-1 and $CD8^+$ CTL-2) notably dissolved $RT1.A^1$/TARS-1 cell but not TARS-1 cells, however, dissolution was not recognized in $CD4^+$ CTL cell lines derived from nu/+ rats immunized with FPM1-V1AX cells. These findings show that the cytotoxic activity of $CD8^+$ CTL cell lines specific to nu/+ rat-derived HTLV-I Tax is restrained by $RT1.A^1$ of rat MHC class I.

Example 7 (Identification of Recognition Epitope of HTLV-I-Specific CTLs)

Peptide mapping analysis was conducted to identify target epitopes recognized by HTLV-I Tax-specific $CD8^+$ CTLs obtained from nu/+ rats immunized with FPM1-V1AX. HTLV-I-specific CTL cell lines restrained by rat MHC class I ($RT1.A^1$) were established by repeating stimulation with formalin-fixed FPM1-V1AX every two weeks to splenic T cells derived from syngeneic immunocompetent rats immunized with nu/+ rat-derived HTLV-I-infected cell lines FPM1-V1AX. Some of a series of synthetic peptides corresponding to the amino acid sequences of HTLV-I Tax shown in Figs. 6, 7 and 8 were synthesized using a solid phase peptide synthesis method on an automated peptide synthesizer (model PSSM-8; Shimazu Corporation, Kyoto, Japan) with chemicals and program cycles provided by the manufacturer, then separated from plastic using hydrogen fluoride, and purified to on and above 95% purity by a reverse-phase chromatography on an HPLC system (Model SPRINT; PE Biosystems Japan, Tokyo, Japan). In addition, 9 mer oligopeptides (Tax 179-187, 180-188, 181-189, 182-190, 183-191, 184-192, 185-193, 186-194, 187-195, and influenza Matrix 58-66) were synthesized by Hokudo Co. (Hokkaido, Japan) on commission and used herein.

For sensitization of the target cells to be used for the cytotoxic activity analysis, 29 partially overlapping synthetic peptides (15-24 mers; partial peptide in the Tax proteins shown in Figs. 6 and 7) were added to target cells derived from syngeneic rats labeled with a radioactive isotope $[^3H]$-TdR (G14 cells; 5 x $10^3$ cells/well) to a concentration of 10μM each, and cultured at 37° C for 1 hour, then the sensitivity for HTLV-I-specific CTL cell lines was measured by $[^3H]$-TdR release assay for 6 hours. Values (mean ± SD) were evaluated from three independent experiments. In addition, E/T was set at 10. The results are shown in Figs. 6 and 7. These results show that the HTLV-I-specific CTL cell lines effectively dissolve target cells sensitized with peptide Tax 177-200 or Tax 181-195, and show low cytotoxic activity against target cells sensitized with peptide Tax 17-40, Tax 33-56, Tax 81-104, or Tax 97-120 (Fig. 6). It was confirmed that long-term-cultured CTL cell lines also notably dissolved target cells sensitized with Tax 177-200 or Tax 181-195 (Fig. 7). Significant cytotoxicity was not recognized in target cells sensitized with synthetic peptides other than Tax 177-200 or Tax 181-195.

Based on the above-mentioned results, 10 different types of 9 amino acid synthetic peptides (9m peptide) included in the region of Tax 177-200 were synthesized, and detailed mapping of HTLV-I Tax-specific CTL epitope was conducted in the same method as mentioned above. The amino acid sequences of synthetic peptides used herein are shown in Table 1, and the results of cytotoxic activity analysis using these 9 amino acid synthetic peptides are shown in Fig. 8. These results show that the target cells sensitized with peptide Tax 180-188, Tax 181-189 show particularly strong CTLs sensitivity, then the target cells sensitized with Tax 179-187, Tax 182-190 exhibited CTL sensitivity for CTLs, however, the effect of Tax 179-187 varies among multiple experiments. The above-mentioned results indicated the possibility of the presence of the dominant epitope recognized by HTLV-I-specific CTLs in Tax 180-188 to Tax 182-190.

Table 1

| Peptide | Amino acid sequence |
|---|---|
| Tax 177-200 | GQLGAFLTNVPYKRIEELLYKISL |
| Tax 181-195 | AFLTNVPYKRIEELL |
| Tax 179-187 | LGAFLTNVP |
| Tax 180-188 | GAFLTNVPY |
| Tax 181-189 | AFLTNVPYK |
| Tax 182-190 | FLTNVPYKR |
| Tax 183-191 | LTNVPYKRI |
| Tax 184-192 | TNVPYKRIE |
| Tax 185-193 | NVPYKRIEE |
| Tax 186-194 | VPYKRIEEL |
| Tax 187-195 | PYKRIEELL |
| Tax 190-198 | RIEELLYKI |
| Tax 11-19 (control peptide) | LLFGYPVYV |

Subsequently, the peptide concentrations of Tax 180-188, Tax 181-189, Tax 182-190 necessary for inducing cytotoxic activity of CTLs were examined in order to clarify the dominant recognition epitope. Target cells G14 treated respectively with serially-diluted peptides for 1 hour in advance, were cocultured with HTLV-I-specific CTL cell lines, and the CTL sensitivities of target cells were examined. The E/T ratio was at 10, and values (mean ± SD) were evaluated from three independent experiments. The results are shown in Fig. 9. These results show that strong CTL sensitivity was confirmed in target cells sensitized with Tax 180-188 (●), and they show sensitivity to CTL even in extremely low peptide concentration, i.e. $10^{-3}$pM. On the other hand, it was revealed that the concentration of 1μM for Tax 181-189 (■), and 10μM for Tax 182-190 (▲) were necessary to induce cytotoxic activity of the same extent as Tax 180-188. No CTL sensitivity was recognized at any concentration in Tax 11-19 (○) which was used as a control therein. The above-mentioned results revealed that the dominant recognition epitope of HTLV-I-specific CTLs restrained by $RT1.A^1$ was a peptide comprising 9 amino acids, i.e. Tax 180-188 (GAFLTNVPY: Seq. ID No.2).

Example 8 (Recognition of Tax 180-188 by Tax-specific CTLs of HTLV-I)

In order to examine whether the peptide Tax 180-188 is dominant among the target epitopes in HTLV-I Tax recognized by HTLV-I-specific CTL cell lines, the cytotoxic activities of HTLV-I Tax-specific CTL cell lines against [$^3$H]-TdR-labeled G14-Tax cell were measured in the presence of G14 cells and unlabeled G14-Tax cells (●) which had been sensitized in advance with unlabeled competitors such as, untreated G14 cells (○), 10 μM of Tax 180-188 (■), or Tax 11-19 (▲). The E/T ratio was at 10, and values (mean ± SD) were evaluated from three independent experiments. The results are shown in Fig. 10. As a result, it was revealed that when G14 cells treated with 10 μM of Tax 180-188 (■) were used, the cytotoxic activities against radiolabeled G14-Tax were completely inhibited when the competitor/Target cells ratio was at 40. The competitive effect of G14 cells treated with Tax 180-188 was at the same extent as the competitive effect of unlabeled G14-Tax (●). Untreated G14 cells (○) or G14 cells treated with 10 μM of various peptides [Tax 181-189, Tax 182-190, and Tax 11-19 (▲)], scarcely affected the cytotoxic activities of HTLV-I Tax-specific CTL cell lines. These results indicate that the epitope Tax 180-188 is the dominant epitope recognized by HTLV-I Tax-specific CTL cell lines.

Example 9 (Suppression Effect on Proliferation of HTLV-I-infected Tumor Cells of Tax 180-188 Recognition CTL cell lines In Vivo)

It was examined whether Tax 180-188 recognition CTL cell lines can possibly suppress the proliferation of HTLV-I-infected tumor cell FPM1-V1AX in rats in vivo. Suppression effect on tumor proliferation in rats wherein FPM1-V1AX was inoculated subcutaneously and CTL cell lines ($10^7$) that recognize Tax 180-188 were simultaneously administered intraperitoneally (▲) was observed by using nu/nu female rats subcutaneously inoculated with HTLV-I-infected tumor cell FPM1-V1AX (2 x $10^7$) alone as positive controls. Besides, the change of tumor proliferation was measured by measuring volume of each subcutaneous tumor by a caliper every other day as in Example 3, and the tumor volumes (V;$mm^3$) were thus measured. Time courses of these subcutaneous tumor proliferations are shown in Fig. 11. The results show that subcutaneous tumors continued to proliferate in rats inoculated with FPM1-V1AX alone (●), whereas such proliferation was promptly rejected in rats simultaneously transfected with CTLS (▲). As a result of the above-mentioned, it was indicated that Tax 180-188 recognition CTL cell lines can possibly suppress in vivo proliferation of HTLV-I-infected tumor cells, in other words, there is a possibility that Tax 180-188 peptide is an important epitope as a tumor rejection antigen.

Example 10 (Vaccine Effect of Tax 180-188 and ISS-ODN)

Whether the antigen peptide determined as above-mentioned has actually become a tumor rejection antigen alone in vivo is extremely important in considering to what extent this antigen peptide can be applied as a tumor vaccine model. Subsequently, therefore, it was considered whether a peptide vaccine can induce Tax 180-188-specific CTLs in vivo, and whether such induced CTLs can show an anti-tumor effect in vivo. ISS-ODN (Immunostimulatory DNA sequences-oligodeoxynucleotide) containing CpG motif was used as an adjuvant, in order to induce Tax 180-188-specific CTLs more efficiently (Nat. Med. 3, 849-854, 1997). In addition, as an ISS-ODN [ESPEC OLIGO SERVICE; 5'-TGACTGTGAACGTTCGAGATGA-3' (Seq. ID No.5)], the one synthesized as phosphorothioate single-stranded oligonucleotides were used. 100μg of Tax 180-188 synthetic peptide (■), 10 nmol of ISS-ODN(▲), 100 μ g of Tax 180-188 mixed with 10 nmol of ISS-ODN (*), or 100 μ g of Influenza A matrix (GILGFVFTL; Seq. ID No.6) peptide comprising the 58-66$^{th}$ amino acid sequences mixed with 10 nmol of ISS-ODN( ♦ ) were respectively mixed in 200 μl of phisiological saline and administered subcutaneously into 4-week-old nu/+ female rats, and they were immunized again after two weeks on the same condition. Besides, rats subcutaneously administered with physiological saline alone were used as controls (●). Two weeks after the last immunization, splenic T cells ($10^7$) were separated from each rat, then administered intraperitoneally to 4-week-old nu/nu female rats, that were simultaneously administered intraperitoneally with HTLV-I-infected tumor cells, FPM1-V1AX (2 x $10^7$). The suppression effect on tumor proliferation at the inoculated sites were measured in the same manner as Example 3, i.e. by measuring the size of each subcutaneous tumor by a caliper every other day, thus the volumes of tumors were measured (V; $mm^3$).

The results of the above are shown in Fig. 12. As a result, the suppression effect on tumor proliferation was strongly suppressed in a group wherein splenic T cells derived from rats immunized with Tax 180-188 and ISS-ODN (*) were transfected, however, such suppression effect on tumor proliferation was not recognized in the group transfected with splenic T cells derived from rats immunized respectively with the following: Tax 180-188 peptide only (■); ISS-ODN only (▲); Influenza A matrix 58-66 and ISS-ODN(♦). As a result of the above-mentioned, it is revealed to be possible to induce Tax 180-188-specific CTLs to immunocompetent rats by using an appropriate adjuvant, and that such induced CTLs can strongly suppress the proliferation of HTLV-I-infected tumor cells in vivo. These results show that Tax 180-188 can act independently as a tumor rejection antigen, and that a vaccine wherein Tax 180-188 peptide and ISS-ODN are used together efficiently induce T cell immunity and protect from HTLV-I-infected tumors in vivo, and that the present experimental system is extremely useful as a developmental model of a vaccine.

**Industrial Applicability**

CTLs induced by using protein, peptide, nucleic acid and the like of a CTL recognition antigen or an atigen epitope thereof which can induce CTLs having an anti-tumor effect against HTLV-I tumors as an immunogen, and by using an adjuvant obtained from the screening method of the present invention can strongly suppress the proliferation of HTLV-I-infected tumor cells in vivo. Therefore, protein, peptide, nucleic acid and the like of such CTL recognition antigen or such antigen epitope thereof which can induce CTLs having an anti-tumor effect against HTLV-I tumors are useful as a vaccine for HTLV-I-associated diseases such as an adult T cell leukemia (ATL) , etc. , and CTLs induced by said protein, peptide, nucleic acid and the like are useful as a preventive and therapeutic agent for the above-mentioned HTLV-I-associated diseases.

## Claims

1. A screening method for a CTL recognition antigen or an antigen epitope thereof which can induce cytotoxic T lymphocytes (CTLs) having an anti-tumor effect against HTLV-I tumors, wherein CTLs induced by a test substance is administered to a non-human animal model of HTLV-I-associated disease, and the change of tumors in said non-human animal is measured and assessed.

2. The screening method for a CTL recognition antigen or an antigen epitope thereof which can induce CTLs having an anti-tumor effect against HTLV-I tumors according to claim 1, wherein the non-human animal model of HTLV-I-associated disease is a non-human animal model of adult T cell leukemia.

**3.** The screening method for a CTL recognition antigen or an antigen epitope thereof which can induce CTLs having an anti-tumor effect against HTLV-I tumors according to claim 1 or 2, wherein the non-human animal is a rat.

**4.** A screening method for a CTL recognition antigen or an antigen epitope thereof which can induce CTLs having an anti-tumor effect against HTLV-I tumors, wherein CTLs induced by a test substance is contacted with HTLV-I-infected tumor cell lines, and the cytotoxic activity of said CTLs is measured and assessed.

**5.** A screening method for a CTL recognition antigen or an antigen epitope thereof which can induce CTLs having an anti-tumor effect against HTLV-I tumors, wherein target cells sensitized with a test substance or target cells that express a test substance are contacted with HTLV-I-specific CTL cell lines, and the cytotoxic activity of said HTLV-I-specific CTL cell lines is measured and assessed.

**6.** The CTL recognition antigen or the antigen epitope thereof which can induce CTLs having an anti-tumor effect against HTLV-I tumors, which is obtained by the screening method according to any of claims 1 to 5.

**7.** The antigen epitope according to claim 6, wherein the antigen epitope is a peptide comprising an amino acid sequence shown by Seq. ID No.2.

**8.** A screening method for an adjuvant that enhances an anti-tumor effect against HTLV-I tumors, wherein CTLs induced by using the CTL recognition antigen or the antigen epitope thereof according to claim 6 and a test adjuvant are administered to a non-human animal model of HTLV-I-associated disease and the change of tumors in said non-human animals is measured and assessed.

**9.** The screening method for an adjuvant that enhances an anti-tumor effect against HTLV-I tumors according to claim 8, wherein the non-human animal model of HTLV-I-associated disease is a non-human animal model of adult T cell leukemia.

**10.** The screening method for an adjuvant that enhances an anti-tumor effect against HTLV-I tumors according to claim 8 or 9, wherein the non-human animal is a rat.

**11.** A screening method for an adjuvant that enhances an anti-tumor effect against HTLV-I tumors, wherein CTLs induced by using the CTL recognition antigen or the antigen epitope thereof according to claim 6 and a test adjuvant are contacted with HTLV-I-infected tumor cell lines, and the cytotoxic activity of said CTLs is measured and assessed.

**12.** The screening method for an adjuvant that enhances an anti-tumor effect against HTLV-I tumors according to any of claims 8 to 11, wherein the antigen epitope peptide according to claim 7 is used as the CTL recognition antigen or the antigen epitope thereof according to claim 6.

**13.** A vaccine for inducing an immune response containing a CTL recognition antigen or an antigen epitope thereof which can induce CTLs having an anti-tumor effect against HTLV-I tumors and which can be obtained by the screening method according to any of claims 1 to 5 as an active ingredient.

**14.** A vaccine for inducing an immune response containing a DNA that encodes a CTL recognition antigen or an antigen epitope thereof which can induce CTLs having an anti-tumor effect against HTLV-I tumors obtained by the screening method according to any of claims 1 to 5 as an active ingredient.

**15.** The vaccine for inducing an immune response according to claim 13 or 14, wherein the CTL recognition antigen is an HTLV-I Tax protein shown by Seq. ID No.1.

**16.** The vaccine for inducing an immune response according to claim 13 or 14, wherein the CTL recognition antigen is a protein comprising an amino acid sequence wherein one or a few amino acids are deleted, substituted, or added in the amino acid sequence shown by Seq. ID No.1, which can induce CTLs having an anti-tumor action against HTLV-I tumors.

**17.** The vaccine for inducing an immune response according to claim 13 or 14, wherein the CTL recognition antigen epitope is a peptide comprising the amino acid sequence shown by Seq. ID No.2.

**18.** The vaccine for inducing an immune response according to claim 13 or 14, wherein the CTL recognition antigen epitope is a peptide comprising an amino acid sequence wherein one or a few amino acids are deleted, substituted, or added in the amino acid sequence shown by Seq. ID No . 2 , which can induce the CTLs having an anti-tumor action against HTLV-I tumors.

**19.** The vaccine for inducing an immune response according to any of claims 13 to 18, further containing an adjuvant that enhances an anti-tumor effect against HTLV-I tumors.

**20.** The vaccine for inducing an immune response according to claim 19, wherein the adjuvant which can be obtained by the screening method according to any of claims 8 to 12 is an adjuvant that enhances an anti-tumor effect against HTLV-I tumors.

**21.** The vaccine for inducing an immune response according to claim 20, wherein the adjuvant that enhances an anti-tumor effect against HTLV-I tumors which can be obtained by the screening method according to any of claims 8 to 12 is ISS-ODN.

**22.** An HTLV-I recognition CTL induced by the vaccine for inducing an immune response according to any of claims 13 to 21.

**23.** A pharmaceutical composition containing the HTLV-I recognition CTL according to claim 22 as an active ingredient.

FIG 1

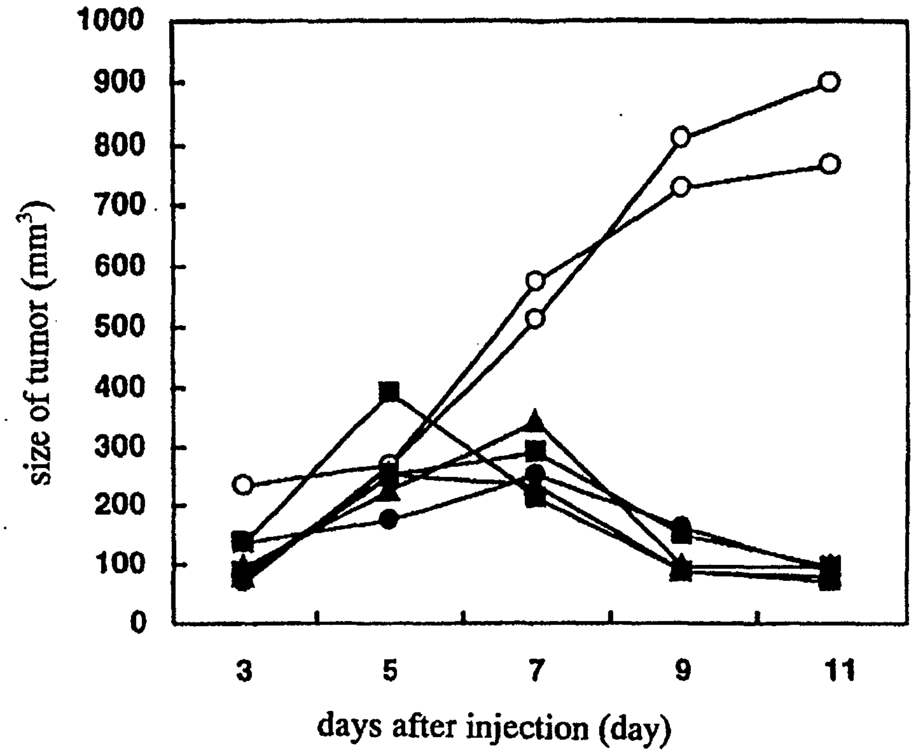
1000
900
800
700
600
500
400
300
200
100
0
size of tumor (mm3)
3
5
7
9
11
days after injection (day)

FIG 2

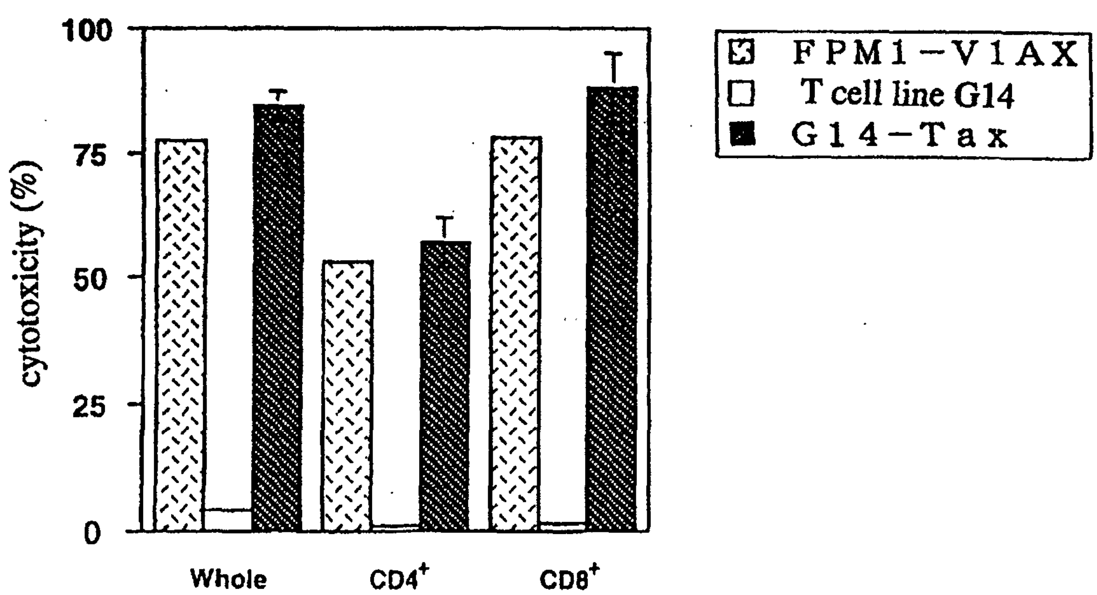
100
75
50
25
0
cytotoxicity (%)
Whole
CD4+
CD8+
FPM1−V1AX
T cell line G14
G14−Tax

FIG 3

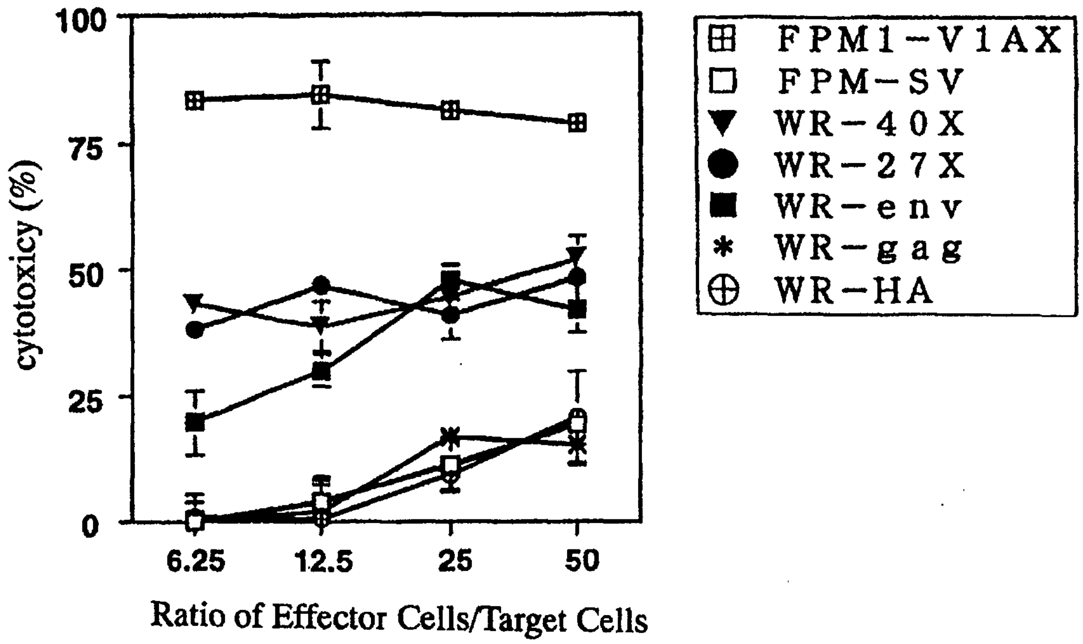
100
75
50
25
0
cytotoxicy (%)
6.25
12.5
25
50
Ratio of Effector Cells/Target Cells
FPM1-V1AX
FPM-SV
WR-40X
WR-27X
WR-env
WR-gag
WR-HA

FIG 4

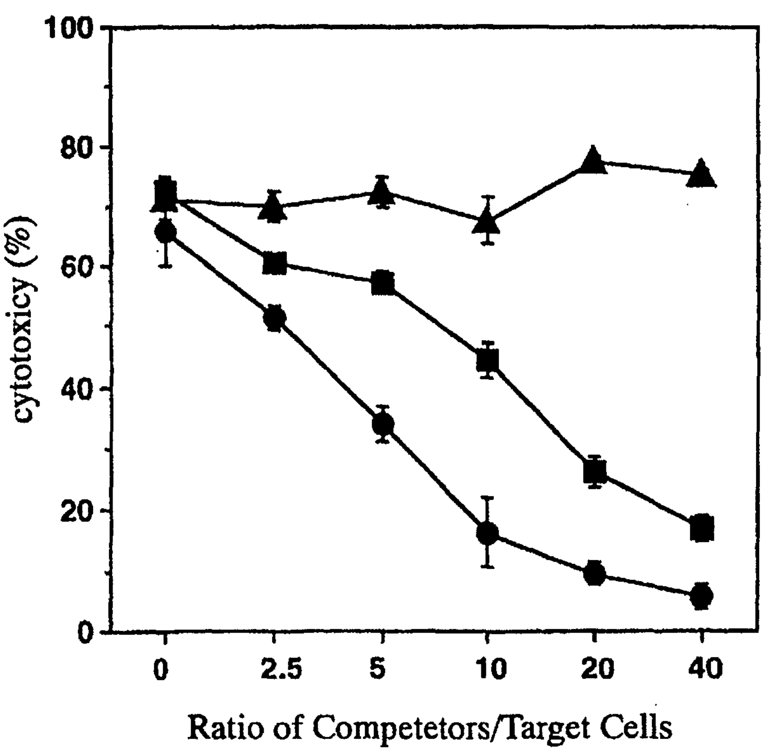
100
80
60
40
20
0
cytotoxicy (%)
0
2.5
5
10
20
40
Ratio of Competetors/Target Cells

FIG 5

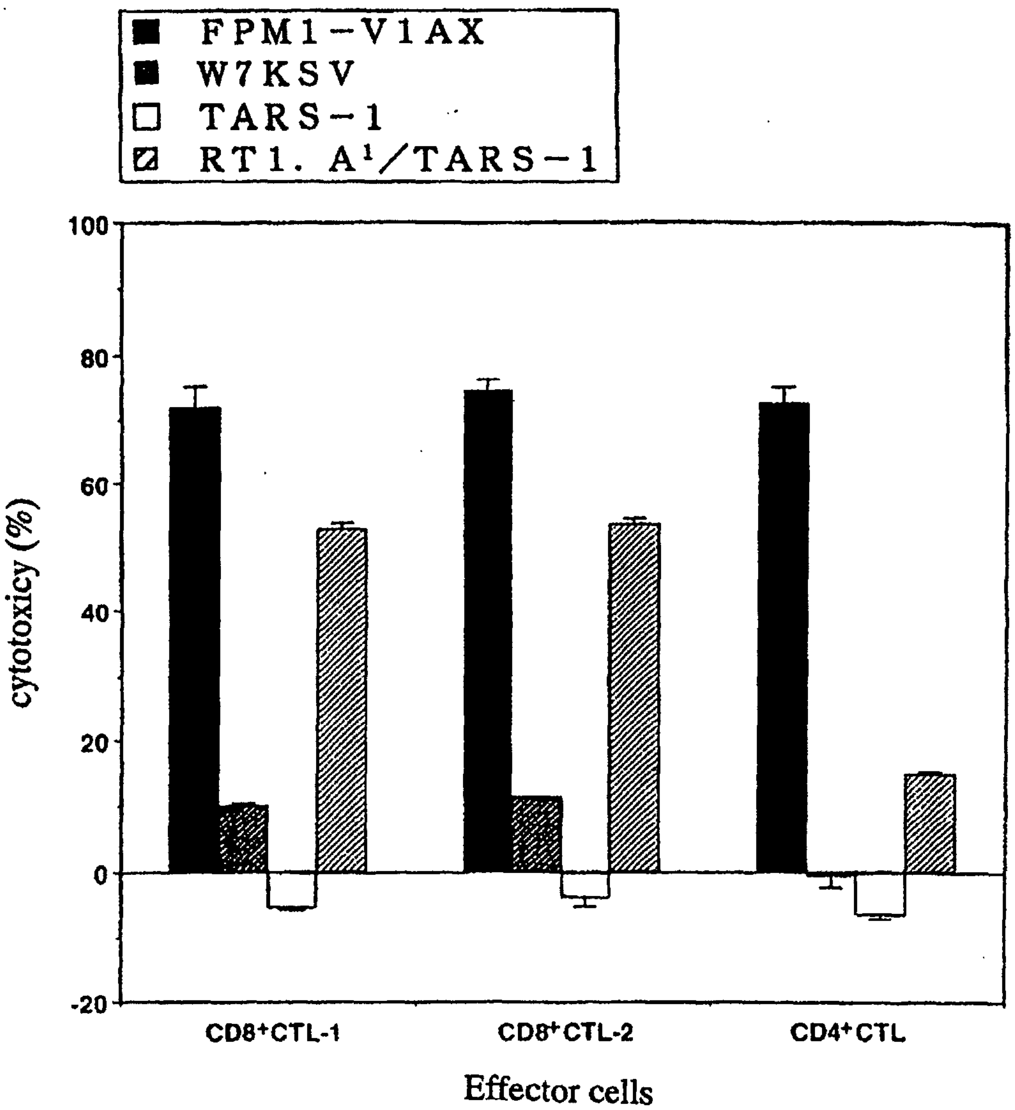
FPM1-V1AX
W7KSV
TARS-1
RT1. A1/TARS-1
100
80
60
40
20
0
-20
cytotoxicy (%)
CD8+CTL-1
CD8+CTL-2
CD4+CTL
Effector cells

FIG 6

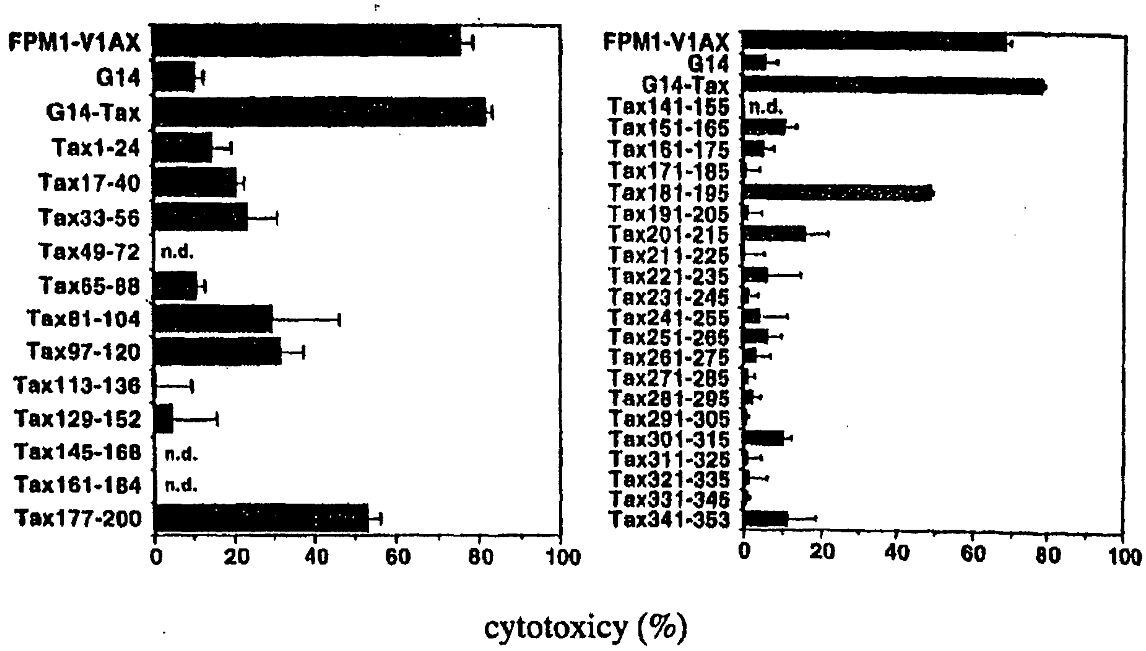
FPM1-V1AX
G14
G14-Tax
Tax1-24
Tax17-40
Tax33-56
Tax49-72
n.d.
Tax65-88
Tax81-104
Tax97-120
Tax113-136
Tax129-152
Tax145-168
n.d.
Tax161-184
n.d.
Tax177-200
0 20 40 60 80 100
FPM1-V1AX
G14
G14-Tax
Tax141-155
n.d.
Tax151-165
Tax161-175
Tax171-185
Tax181-195
Tax191-205
Tax201-215
Tax211-225
Tax221-235
Tax231-245
Tax241-255
Tax251-265
Tax261-275
Tax271-285
Tax281-295
Tax291-305
Tax301-315
Tax311-325
Tax321-335
Tax331-345
Tax341-353
0 20 40 60 80 100
cytotoxicy (%)

FIG 7

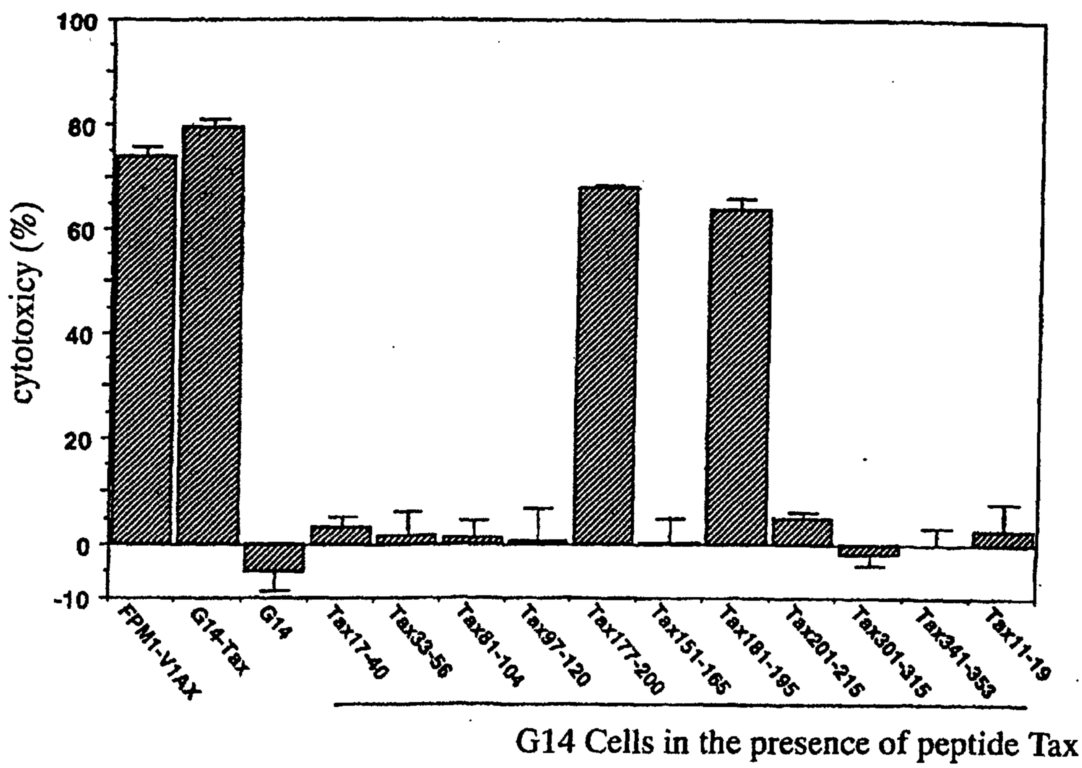
cytotoxicy (%)
100
80
60
40
20
0
-10
FPM1-V1AX
G14-Tax
G14
Tax17-40
Tax33-56
Tax81-104
Tax97-120
Tax177-200
Tax151-165
Tax181-195
Tax201-215
Tax301-315
Tax341-353
Tax11-19
G14 Cells in the presence of peptide Tax

FIG 8

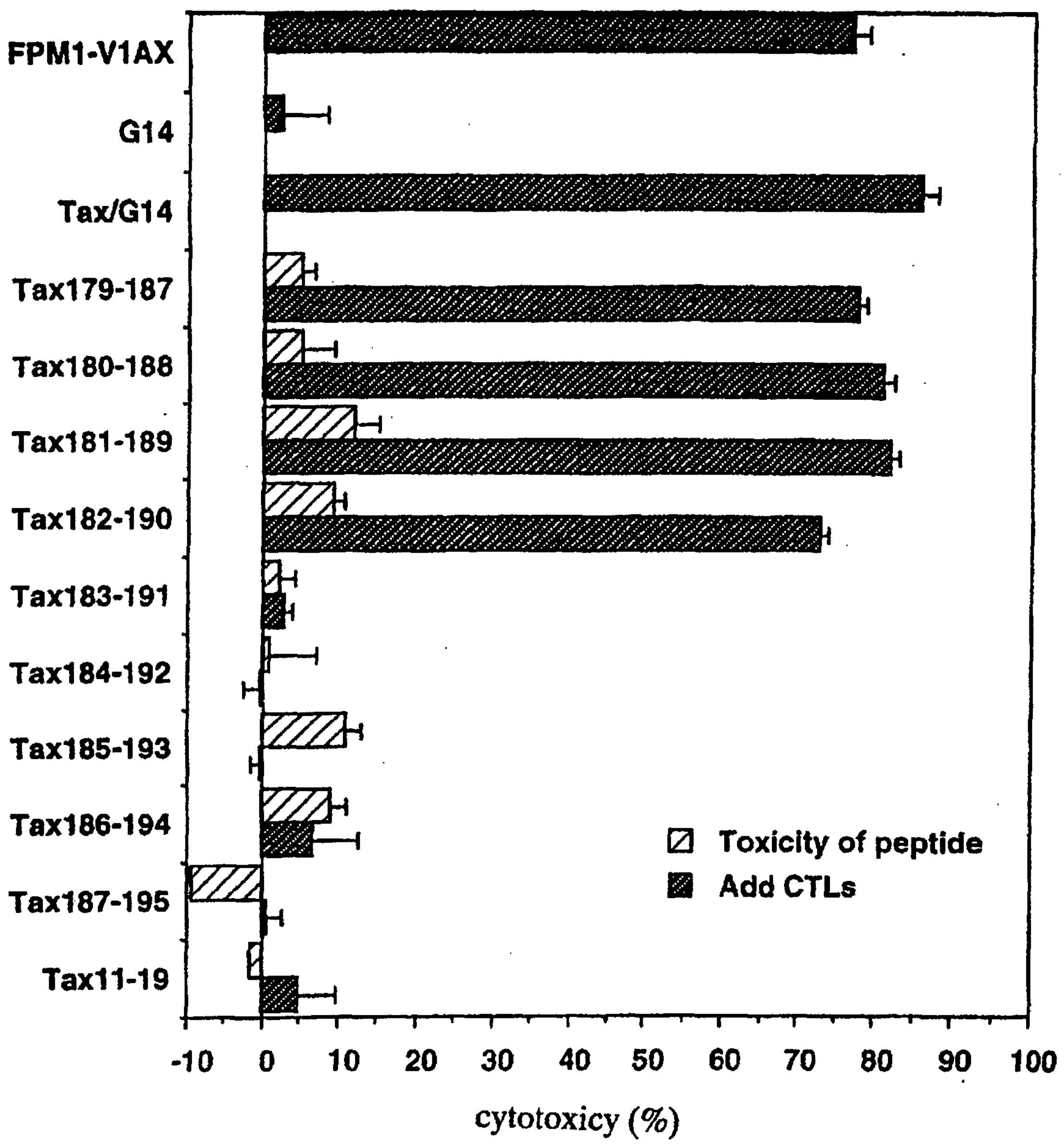
FPM1-V1AX
G14
Tax/G14
Tax179-187
Tax180-188
Tax181-189
Tax182-190
Tax183-191
Tax184-192
Tax185-193
Tax186-194
Tax187-195
Tax11-19
Toxicity of peptide
Add CTLs
-10
0
10
20
30
40
50
60
70
80
90
100
cytotoxicy (%)

FIG 9

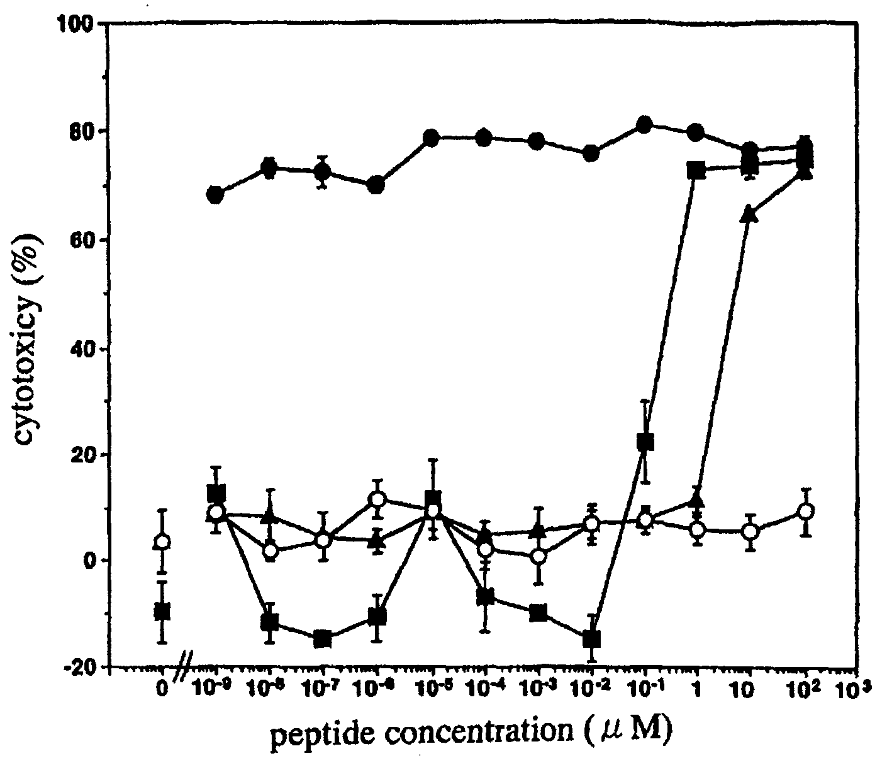
100
80
60
40
20
0
-20
cytotoxicy (%)
0
10-9
10-8
10-7
10-6
10-5
10-4
10-3
10-2
10-1
1
10
10²
10³
peptide concentration (μM)

FIG 10

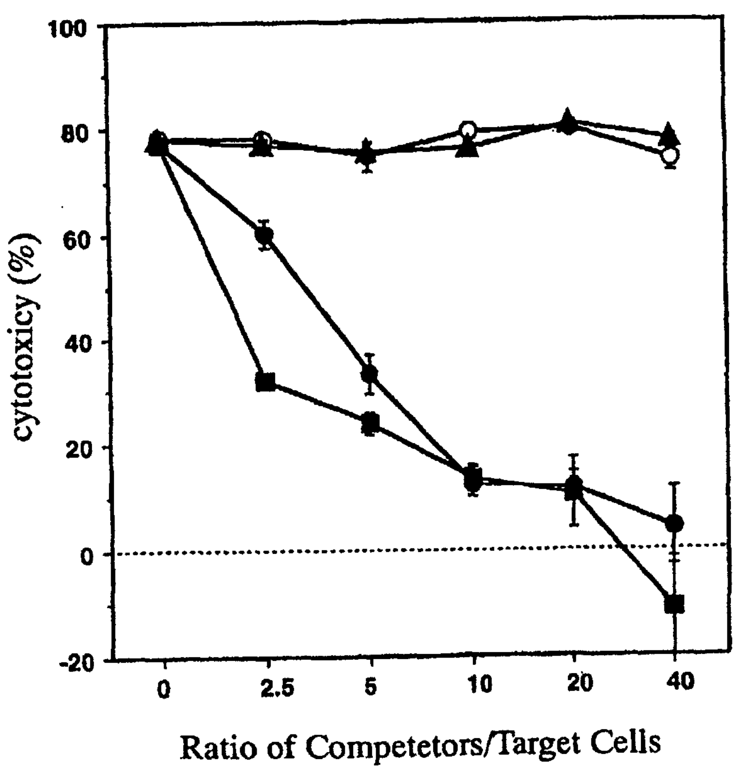
100
80
60
40
20
0
-20
cytotoxicy (%)
0
2.5
5
10
20
40
Ratio of Competetors/Target Cells

FIG 11

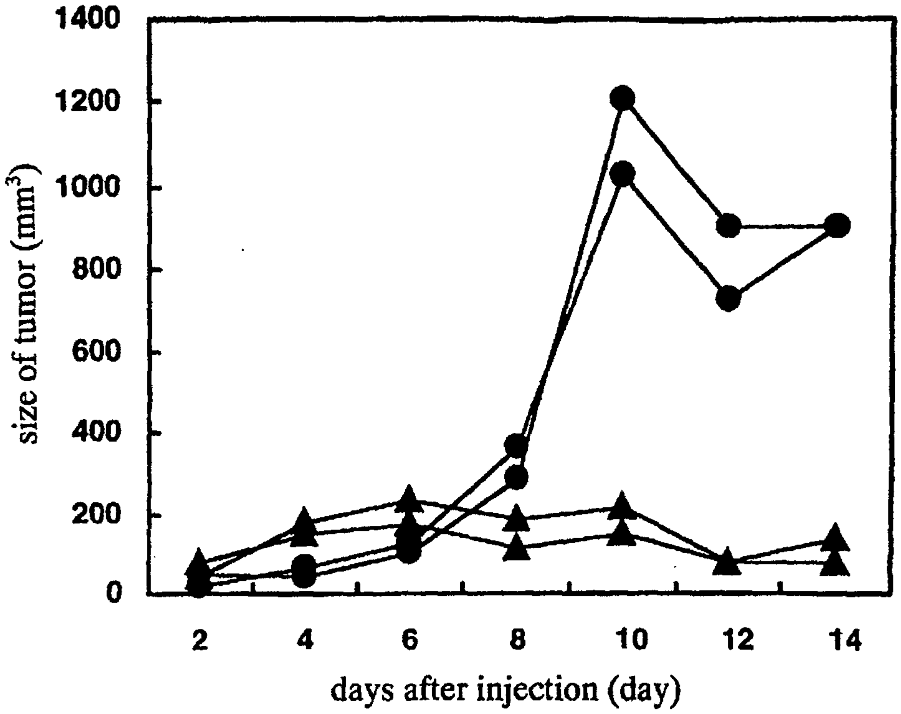
1400
1200
1000
800
600
400
200
0
size of tumor (mm3)
2
4
6
8
10
12
14
days after injection (day)

FIG 12

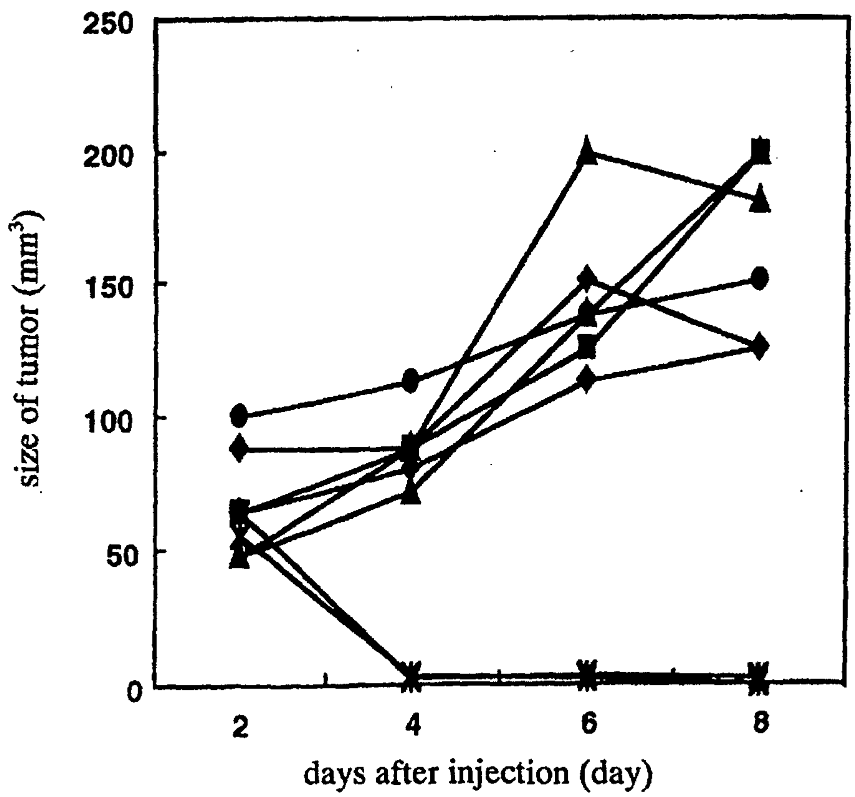
250
200
150
100
50
0
size of tumor (mm3)
2
4
6
8
days after injection (day)

## INTERNATIONAL SEARCH REPORT

International application No.
PCT/JP02/04406

A. CLASSIFICATION OF SUBJECT MATTER
Int.Cl$^7$ G01N33/50, 33/15, A61K39/00

According to International Patent Classification (IPC) or to both national classification and IPC

B. FIELDS SEARCHED

Minimum documentation searched (classification system followed by classification symbols)
Int.Cl$^7$ G01N33/50, 33/15, A61K39/00

Documentation searched other than minimum documentation to the extent that such documents are included in the fields searched

| | | | |
|---|---|---|---|
| Jitsuyo Shinan Koho | 1922-1996 | Toroku Jitsuyo Shinan Koho | 1994-2002 |
| Kokai Jitsuyo Shinan Koho | 1971-2002 | Jitsuyo Shinan Toroku Koho | 1996-2002 |

Electronic data base consulted during the international search (name of data base and, where practicable, search terms used)
BIOSIS, JOIS

C. DOCUMENTS CONSIDERED TO BE RELEVANT

| Category* | Citation of document, with indication, where appropriate, of the relevant passages | Relevant to claim No. |
|---|---|---|
| A | JP 2001-89389 A (Sumitomo Pharmaceuticals Co., Ltd.), 03 April, 2001 (03.04.01), (Family: none) | 1-5,7-12, 15-19,21-23 |
| A | JP 2001-501827 A (Fordham University), 13 February, 2001 (13.02.01), & EP 954564 A | 1-5,7-12, 15-19,21-23 |
| A | WO 00/27186 A (Japan Science and Technology Corp.), 18 May, 2000 (18.05.00), & EP 1127487 A | 1-5,7-12, 15-19,21-23 |
| A | WO 99/02183 A (CTL Immunotherapies Corp.), 21 January, 1999 (21.01.99), & EP 1003548 A | 1-5,7-12, 15-19,21-23 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>05 August, 2002 (05.08.02) | Date of mailing of the international search report<br>20 August, 2002 (20.08.02) |
| Name and mailing address of the ISA/<br>Japanese Patent Office | Authorized officer |
| Facsimile No. | Telephone No. |

Form PCT/ISA/210 (second sheet) (July 1998)

## INTERNATIONAL SEARCH REPORT

International application No.
PCT/JP02/04406

C (Continuation). DOCUMENTS CONSIDERED TO BE RELEVANT

| Category* | Citation of document, with indication, where appropriate, of the relevant passages | Relevant to claim No. |
|---|---|---|
| A | PROC. NATL. ACAD. SCI. U.S.A., Vol.80, No.12, (1983), pages 3618 to 3622 | 1-5,7-12, 15-19,21-23 |

Form PCT/ISA/210 (continuation of second sheet) (July 1998)

## INTERNATIONAL SEARCH REPORT

International application No.
PCT/JP02/04406

**Box I Observations where certain claims were found unsearchable (Continuation of item 1 of first sheet)**

This international search report has not been established in respect of certain claims under Article 17(2)(a) for the following reasons:

1. ☐ Claims Nos.:
because they relate to subject matter not required to be searched by this Authority, namely:

2. ☒ Claims Nos.: 6, 13, 14 20
because they relate to parts of the international application that do not comply with the prescribed requirements to such an extent that no meaningful international search can be carried out, specifically:
It is unclear how far substances are involved in the scopes of the antigen epitopes, vaccines and adjuvants obtained by the screening methods.

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1998)